**Europäisches Patentamt**

**European Patent Office**

**Office européen des brevets**

(19)

(11) Veröffentlichungsnummer: **0 033 732**
**B1**

# EUROPÄISCHE PATENTSCHRIFT

(12)

(45) Veröffentlichungstag der Patentschrift:
07.01.87

(21) Anmeldenummer: **80901410.3**

(22) Anmeldetag: **23.07.80**

(86) Internationale Anmeldenummer:
**PCT/EP 80/00062**

(87) Internationale Veröffentlichungsnummer:
**WO 81/00212 (05.02.81 Gazette 81/4)**

(51) Int. Cl.⁴: **A 61 M 16/00**

(54) **BEATMUNGSGERÄT ZUR REANIMATION VON NEUGEBORENEN.**

(30) Priorität: **24.07.79 DE 2929996**
**21.07.80 DE 3027614**

(43) Veröffentlichungstag der Anmeldung:
**19.08.81 Patentblatt 81/33**

(45) Bekanntmachung des Hinweises auf die Patenterteilung:
**07.01.87 Patentblatt 87/2**

(84) Benannte Vertragsstaaten:
**AT CH DE FR GB LI NL SE**

(56) Entgegenhaltungen:
**DE - A - 2 241 291**
**DE - C - 470 956**
**FR - A - 1 032 303**
**US - A - 4 011 866**

(73) Patentinhaber: **Beyreuther, Gisela, Enthaler Strasse 17,
D-8221 Bergen (DE)**
Patentinhaber: **Beyreuther, Felix (ges. vertreten durch
Beyreuther, Gisela), Enthaler Strasse 17, D-8221 Bergen
(DE)**
Patentinhaber: **Beyreuther, Kristin (ges. vertreten durch
Beyreuther, Gisela), Enthaler Strasse 17, D-8221 Bergen
(DE)**
Patentinhaber: **Beyreuther, Moritz (ges. vertreten durch
Beyreuther, Gisela), Enthaler Strasse 17, D-8221 Bergen
(DE)**
Patentinhaber: **Beyreuther, Susann (ges. vertreten durch
Beyreuther, Gisela), Enthaler Strasse 17, D-8221 Bergen
(DE)**

(72) Erfinder: **Beyreuther, Christian, verstorben, (DE)**
Erfinder: **RIEGEL, Klaus, Veilchenstrasse 21a,
D-8000 München 21 (DE)**

(74) Vertreter: **Körber, Wolfhart, Dr. et al, Patentanwälte
Dipl.-Ing. H. Mitscherlich Dipl.-Ing. K. Gunschmann
Dr.rer.nat. W. Körber Dipl.Ing. J. Schmidt-Evers Dipl.-Ing.
W. Melzer Steinsdorfstrasse 10,
D-8000 München 22 (DE)**

ACTORUM AG

## Beschreibung

Die Erfindung betrifft ein Beatmungsgerät zur Reanimation von Neugeborenen, mit einem Wasserschloss als Einatemventil mit einem mit dem Steigrohr des Wasserschlosses verbundenen T-Verbindungsstück, durch das das Wasserschloss über Atemschläuche mit einer Atemgasquelle und mit einem Patientenadapter verbunden ist, der eine mit der Atmosphäre in Verbindung stehende verschliessbare freie Öffnung für die Ausatmung besitzt, bei dem die Menge des von der Atemgasquelle zugeführten Atemgases durch einen Regler einstellbar ist, bei dem die maximale Eintauchtiefe des Steigrohrs den maximalen inspiratorischen Beatmungsdruck bestimmt und bei dem die Verschlussdauer der Ausatemöffnung Atemfrequenz und Atemphase bestimmt.

Das wichtigste Ereignis nach der Geburt ist das Ingangkommen des pulmonalen Gaswechsels, das heisst, die eigenständige Lungenatmung (Spontanatmung) des Neugeborenen. Die verlängerte primäre Apnoe (Atemstillstand) ist der wichtigste Zwischenfall. Sie beruht auf einer Lähmung des Atemzentrums durch Sauerstoffmangel während der Geburt oder durch Narkotika, die der Mutter vor oder während der Geburt verabreicht wurden. Dieser äusserst bedrohliche Zustand muss unverzüglich durch Reanimation behoben werden. Eine nicht rasche Belüftung der Lunge des Neugeborenen hat ernste Anpassungs-und Entwicklungsstörungen für das Kind zur Folge. In der Regel führt in diesem akut bedrohlichen Zustand eine sofortige, jeweils 10–15 Sek. dauernde initiale Blähung der Lunge mit komprimiertem Sauerstoff, eine sogenannte Blähdruckbeatmung, über eine Entfaltung der Alveolen schnell zur gewollten Spontanatmung.

Insbesondere bei unreifen Neugeborenen (Frühgeborenen) muss nicht selten die Atmung zusätzlich über längere Zeit unterstützt werden. Ein wesentliches Zeichen der Unreife ist der Mangel an ausreichender Menge oberflächenaktiver Substanz, das sogenannte Surfactant-Mangel-Syndrom oder idiopatische Atemnotsyndrom. Die Folge ist, dass bei jeder Ausatemphase (Exspiration), aufgrund der ungenügend verminderten Oberflächenspannung, Alveolen kollabieren. Kollabierte Alveolen können aber am Atemgaswechsel nicht mehr teilnehmen. Das Blut wird mit Sauerstoff untersättigt. Dieser Sauerstoffmangel führt in zunehmendem Mass zur Atemnot und schliesslich durch Lähmung des Atemzentrums zum Atemstillstand, wenn nicht rechtzeitig verhindert wird, dass der Kollapsdruck in den Alveolen unterschritten wird. Zur Unterstützung der Atmung beim idiopatischen Atemnotsyndrom hat sich daher in letzter Zeit mit grossem Erfolg ein Beatmungsverfahren bewährt, das ständig einen kontinuierlichen positiven Druck (CPAP) bis zu 1,5 kPa (15 cm $H_2O$) in den Luftwegen aufrechterhält, der dem Alveolarkollaps entgegengerichtet ist, die sogenannte CPAP-Beatmung. Eine frühzeitige, unmittelbar nach der Geburt erfolgende, konsequente Anwendung dieser Methode erhält die Spontanatmung aufrecht und erspart solchen Kindern eingreifendere Beatmungstechniken mit deren nicht unerheblichen Nebenwirkungen.

Zeigt das Neugeborene trotzdem Zeichen zunehmender Atemnot, muss der Atemzyklus zeitweilig mit einem Beatmungsgerät künstlich nachgeahmt werden. Dabei wird periodisch in der Einatemphase (Inspiration) ein positiver Druck zu deren Blähung erzeugt (IPPV) und in der Ausatemphase ein positiver endexspiratorischer Druck (PEEP) oberhalb des Kollapsdrucks der Alveolen aufrechterhalten; es wird die sogenannte IPPV + PEEP-Beatmung durchgeführt. Die untere Druckgrenze (PEEP) entspricht hierbei der bei der CPAP-Beatmung, die obere aber nicht der zur initialen Blähdruckbeatmung erforderlichen. Die initiale Entfaltung der Lunge verlangt nämlich generell einen höheren Beatmungsdruck, als er zur Unterhaltung der Atmung notwendig ist.

Von einem Gerät zur Beatmung von Neugeborenen mit Einsatzschwerpunkt in Entbindungskliniken und beim Transport in eine Kinderklinik ist zu fordern, dass es in einfacher und zuverlässiger Weise diesen Erfordernissen einer Beatmung Neugeborener kontrollierbar angepasst werden kann. Verzichtet werden kann auf eine Automatisierung der Beatmungsvorgänge, da zum einen die initiale Blähdruckbeatmung, den individuellen Bedürfnissen angepasst, von Hand vorzunehmen ist bzw. die CPAP-Beatmung nach stabilisierter Eigenatmung keinerlei Steuerung mehr bedarf und zum anderen der nach der Geburt noch äusserst instabile Kreislauf des Neugeborenen eine individuelle und sich an den rasch ändernden Verhältnissen orientierende flexible Therapie verlangt, die am besten unmittelbar durch Handbeatmung gewährleistet ist. Die automatisch gesteuerte Beatmung bei respiratorischer Insuffizienz ist dagegen erst in der stationären Behandlung unerlässlich. Weiter muss unkomplizierter Aufbau und leichte Einstellung des Beatmungsgerätes zur Vermeidung ungewollter Fehlbedienungen gefordert werden, sollen auch in der Beatmungstechnik von Kleinkindern Ungeübte damit risikoarm umgehen können. Ein erfahrener Kinderarzt steht auch heute noch selten zur Verfügung. Eine der grössten Gefahren droht dem Neugeborenen nämlich von einem falsch eingestellten Beatmungsdruck durch Lungenriss mit Pneumothorax. Als relativ ungefährliche inspiratorische Beatmungsgrenzdrücke gelten solche bis zu maximal 3 kPa (30 cm Wassersäule ($H_2O$)). Zur initialen Entfaltung der Lunge können jedoch Blähdrücke bis zu 7 kPa (70 cm $H_2O$) notwendig werden, während umgekehrt bei vorgeschädigter Lunge ein Druck von 3 kPa (30 cm $H_2O$) schon zum gefürchteten Lungenriss führen kann. Das bedeutet, dass sich der Behandelnde schrittweise an den individuell erforderlichen und noch tolerierbaren Beatmungsdruck herantasten muss, der die kritische Schlüsselgrösse in der Beatmung Neugeborener darstellt. Seine einfache, fehlerfreie und kontrollierbare Einstellung muss sichergestellt sein. Alle anderen Beatmungsparameter sind dagegen fürs erste von zweitrangiger Bedeutung.

Es sind Beatmungsgeräte für Neugeborene mit automatischer Zeitsteuereinrichtung bekannt (DE-A-2 405 955), die auch von Hand bedient werden können. Diese Geräte stehen mit dem Patienten über zwei, das Atemgas zu- bzw. rückführende Atemschläuche in Verbindung. Beide Atemschläuche münden unmittelbar vor dem Patienten in einen Patientenadapter zum wahlweisen Anschluss einer Atemmaske oder eines Luftröhrentubus (Trachealtubus). Das Atemgas fliesst dabei von einer Druckgasquelle über ein Einatemventil zum Patienten und von ihm über ein Ausatemventil nach aussen in die Atmosphäre. Der inspiratorische Beatmungsdruck wird am Einatemventil eingestellt und geregelt, der exspiratorische Beatmungsdruck, die Atemfrequenz und die Atemphasen am Ausatemventil. Besonderer Wert ist hier auf die zeitgerechte Ansteuerung des Ausatemventils mit einstellbaren Atemphasen gelegt. Die Zeitsteuereinrichtung des Ausatemventils besteht dabei aus einem System pneumatischer oder elektronischer Bauteile. Nachteilig daran sind die mit der technisch sehr aufwendigen Bauweise verbundenen hohen Herstellungs- und Wartungskosten sowie der unwirtschaftlich hohe Gasverbrauch. Ausserdem ist die Bedienung dieser Geräte kompliziert und erfordert viel Erfahrung, da der inspiratorische Beatmungsdruck am Einatemventil nicht voreingestellt werden kann. Der tatsächlich auf die Atemwege einwirkende Druck ist nämlich zusätzlich von der eingestellten Einatemzeit und dem Atemgasstrom abhängig. Ein Eingriff in diesen sensiblen Regelkreis ist problematisch. Um unnötige Gefahren vom Patienten abzuwenden, muss daher vor jeder Umstellung des Gerätes, insbesondere vor Handbeatmung, der am Einatemventil eingestellte Druck zurückgenommen werden, um dann nach erfolgter Umstellung bei geschlossenem Schlauchsystem anhand des Beatmungsdruckmessers neu eingestellt zu werden. Dieses Verfahren ist sehr umständlich und zeitraubend. Eine PEEP-Beatmung ist oft, z.B. bei einer Ausführungsform, die gerade zur Beatmung Neugeborener beim Transport im Handel angeboten wird (Dräger-Prospekt Nr. 5673.0), mangels eines kontinuierlichen exspiratorischen Atemgasstromes nur mit Hilfe geeigneter Zusätze möglich (vgl. DE-B-2 241 291 und DE-B-2 406 679). Aus den angeführten Gründen konnten sich diese Beatmungsgeräte nur auf kinderärztlichen Intensivstationen mit hoher Einsatzfrequenz und entsprechend geschultem Fachpersonal durchsetzen.

Bei einem anderen bekannten manuell oder automatisch steuerbaren Beatmungsgerät (DE-A-2 603 063) wird der Beatmungsdruck unmittelbar im Patientenadapter durch ein Gasstrahlrohr dynamisch erzeugt, dem Atemgas unter einem Druck von 25–250 kPa (0,25 bis 2,5 atü) zugeführt wird. Die dem Trachealtubus gegenüberliegende Seite des Patientenadapters ist zur Atmosphäre hin offen, wobei der Strömungsquerschnitt der Öffnung gleich oder grösser als der Durchflussquerschnitt des Trachealtubus ist, damit während der Beatmung Schleim aus den Luftwegen des Patienten abgesaugt werden kann. Dies ist aber bei Neugeborenen, entgegen den Ausführungen in der genannten Offenlegungsschrift, ohne Unterbrechung des Beatmungsvorgangs infolge sich in etwa entsprechender Querschnitte von Absaugschlauch und Trachealtubus nicht möglich. Der exspiratorisch dynamisch erzeugte Staudruck im Patientenadapter bildet das Ausatemventil. Gesteuert wird die Beatmung abhängig vom Druck im Patientenadapter nahe der Einmündung des Trachealtubus, der mittels eines Fühlers einem Steuergerät zugeführt wird, das über ein Einatemventil die Atemgaszufuhr und die Atemfrequenz nachführt. Bei Ausfall des Steuerventils ist eine Handbeatmung, wie behauptet, nicht möglich, da mit dem Steuergerät die Druckbegrenzung ausfällt und sich ein unkontrolliert hoher Staudruck in der Lunge des Patienten aufbauen kann. Dieses Beatmungsgerät bietet daher gegenüber den vorgenannten keine Vorteile.

Ein bekanntes nur handbetriebenes Atmungsgerät für Kleinkinder stellt der sogenannte Atembeutel dar (Ambu-Prospekt Form 2500.11.75). Er ist preisgünstig und findet daher verbreitete Anwendung. Mit ihm kann zwar eine dem Atemrhythmus entsprechende periodische Beatmung durchgeführt werden, eine Möglichkeit zur Blähdruck-, CPAP- oder IPPV + PEEP-Beatmung fehlt aber. Die Schlüsselgrösse Beatmungsdruck ist unkontrollierbar dem Gefühl des Beatmenden überlassen: Je stärker der Atembeutel mit der Hand komprimiert wird, desto höher wird der in den Atemwegen erzeugte Druck. Bei dem kleinen Atemvolumen Neugeborener ist die angegebene Drucksicherung nicht wirksam. Aus den angeführten Gründen entspricht der Atembeutel bei Beatmung Neugeborener nicht mehr dem heutigen Stand der Beatmungstechnik, sondern ist auch in den Händen Ungeübter gefährlich.

Zur Überwindung der grundsätzlichen vorstehend geschilderten Probleme ist eine manuelle Beatmungseinrichtung (K. Riegel et al. in Dtsch. med. Wschr. 99 (1974), 1624–1626) der eingangs genannten Art bekannt geworden, von der die Erfindung ausgeht. Bei dieser Beatmungseinrichtung ist der inspiratorische Beatmungsdruck über ein als Einatemventil dienendes Wasserschloss kontrollierbar von 0–4 kPa (0–40 cm $H_2O$) den Bedürfnissen Neugeborener einfach anpassbar. Das Wasserschloss ist über ein T-Verbindungsstück zwischen einer Druckgasquelle für Atemgas und dem Patientenadapter angeordnet. Der maximale inspiratorische Beatmungsdruck wird durch die Eintauchtiefe des Steigrohrs in das Wasserniveau begrenzt. Wenn die gemäss dem am Steigrohr vorgegebenem Druck mögliche Blähung der Lunge erreicht ist, entweicht einströmendes Gas vollständig über das Wasserschloss. Ein wesentlicher, gefährlicher Nachteil tritt auf, wenn der zum Wasserschloss führende Schlauch abgeknickt ist, da dann ein unkontrolliert hoher Druck auf die Lunge einwirkt. Der kontinuierliche Atemgasstrom wird unabhängig vom Beatmungsdruck nach Bedarf am Regler für die Atemgaszufuhr voreingestellt. Gesteuert wird die Beatmung über eine freie Öffnung im Patientenadapter, die mit der Atmosphäre in Verbindung steht und der Ausatmung dient. Der Querschnitt dieser Öffnung entspricht dem des Atemschlauches. Atemsynchrones Abdecken dieser Ausatemöffnung mit dem Finger führt zur kontrollierten Beatmung (IPPV). Wird der Finger z.B. 10–15 Sek. auf der Öffnung belassen, ist eine Blähdruckbeatmung erreicht. Dieses Beatmungsgerät stellt, bis auf den genannten Nachteil, ein zuverlässiges System dar, das sich seit Jahren in der Klinik bewährt hat. Es enthält keine sich bewegenden Teile, ist sicher und leicht einzustellen und auch Laien schnell verständlich. Die Schlüsselgrösse Beatmungsdruck ist dabei am Gerät voreinstellbar. Der Anschaffungspreis liegt bei dem des Atembeutels. Nachteilig ist allerdings, dass keine CPAP- sowie IPPV + PEEP-Beatmungen realisierbar sind, ebensowenig wie therapeutisch indizierte initiale Blähdrücke zwischen 4 und 7 kPa (40–70 cm $H_2O$), da bereits bei Drücken von 4 kPa (40 cm $H_2O$) ein Wasserschloss als druckbegrenzendes Medium mit einer dann effektiven Länge von 90 cm unhandlich ist.

Ein im wesentlichen identisches Beatmungsgerät gemäss der US-A-40 11 866 enthält zusätzlich eine elektronische Steuereinrichtung für das Ausatemventil, das selbst nur Schliess-bzw. Öffnungsfunktion besitzt, weshalb keine Möglichkeit besteht, einen positiven endexspiratorischen Beatmungsdruck (PEEP/CPAP) zu erzeugen.

Aus der DE-C-470 956 ist ein einem Wasserschloss entsprechendes Boyle-Mariottsches-Gefäss bekannt, das dort lediglich als Sicherungsglied dient, da das als Steigrohr ausgebildete Luftzuleitungsrohr nicht verschieblich angeordnet ist. Es wirkt als Überdruckventil für Einblasen bzw. als Unterdruckventil für Ausblasen. Der entsprechende Druck ist dabei nur in sehr engen Grenzen veränderbar.

Die FR-A-10 32 303 zeigt eine Atemgas-Insuflationsvorrichtung mit einem Wasserschloss, das als Überdrucksicherung dient und bei dem der Beatmungsdruck nicht einstellbar ist. Das Einatemventil selbst wird durch ein mechanisch arbeitendes federbelastetes Membranventil gebildet, wobei der eingestellte Beatmungsdruck über ein U-Manometer ablesbar ist.

Die DE-A-22 41 291 erläutert eine Zusatzvorrichtung zur Erzeugung positiver endexspiratorischer Beatmungsdrücke in Verbindung mit einem im Handel erhältlichen Beatmungsgerät ohne PEEP/CPAP-Beatmung. Ein dort vorgesehenes einziges Wasserschloss arbeitet als Überdruckventil, über das der kontinuierlich fliessende Leckkompensationsfluss während der Inspirationsstellung eines Einatemventils abfliessen muss. Ein Wasserventil dient zur Erzeugung des positiven endexspiratorischen Drucks während der Ausatemphase.

Auch diese bekannten Beatmungsgeräte vermögen nicht die anhand der Beatmungseinrichtung nach K. Riegel at al, ..., erläuternden Nachteile zu überwinden.

Es ist daher Aufgabe der Erfindung, das bekannte Beatmungsgerät bei einfacher und preisgünstiger Bauweise so auszubilden, dass es den speziellen Bedürfnissen bei der Beatmung Neugeborener unmittelbarer und zuverlässiger gerecht wird.

Die Aufgabe wird durch die kennzeichnenden Merkmale des Anspruchs 1 oder diejenigen des Anspruchs 4 oder diejenigen des Anspruchs 7 gelöst.

Die Erfindung wird durch die Merkmale der abhängigen Ansprüche weitergebildet.

Das erfindungsgemässe Beatmungsgerät ist zweistufig ausgebildet. Die erste Beatmungsstufe besteht aus dem bereits bewährten Wasserschloss, das gleich einem Druckhalteventil das Einatemventil bildet. Dabei ist das T-Verbindungsstück, das das Wasserschloss mit der Atemgasquelle und dem Patientenadapter über zwei Atemschläuche verbindet, vorteilhaft in das Steigrohr starr integriert. Ein Abknicken der Atemschläuche, gleichgültig wo, kann dann nicht mehr zu einem die Lunge gefährdenden unkontrolliert hohen Druck führen. Durch die gewählte Geometrie der Anordnung wird bei geschlossenem Ausatemventil (Inspiration) der zu erzeugende, vom Atemgasstrom unabhängige inspiratorische Staudruck in den Atemwegen auf maximal 3 kPa (30 cm $H_2O$) begrenzt. Die zweite Beatmungsstufe wirkt additiv zur ersten und ist bei der ersten Ausführungsform durch eine nachschaltbare Drosseleinrichtung gebildet, durch die die Gasfüllung vom Wasserschloss abfliessen muss. Auf diese Weise bildet sich vor der Drosseleinrichtung ein Staudruck aus, der allein vom Gasstrom durch die Drosseleinrichtung abhängt und von einem auch als Sicherheitsventil dienenden Flüssigkeitsmanometer auf 4 kPa (40 cm $H_2O$) begrenzbar ist. Mittels der zweiten Beatmungsstufe wird daher ein inspiratorischer Blähdruck der Lunge von bis zu 7 kPa (70 cm $H_2O$) möglich.

Gemäss der zweiten Ausführungsform der Erfindung ist eine zuschaltbare Drosseleinrichtung vorgesehen, die vorzugsweise mit der vorstehend genannten identisch ist, auf die mit der Atmosphäre in Verbindung stehende Ausatem-Öffnung am Patientenadapter aufsteckbar ist und zusammen mit einem Abschlussorgan das Ausatemventil bildet. So kann während der Exspiration ständig ein Staudruck in der Lunge aufrechterhalten werden (CPAP), der wieder allein vom Atemgasstrom durch die letztere Drosseleinrichtung abhängt. Der freie Querschnitt der Drosseleinrichtung ist zweckmässig wesentlich kleiner als der im Trachealtubus.

In einer dritten Ausführungsform der Erfindung ist eine zuschaltbare zweite Beatmungsstufe durch ein Druckbegrenzungsventil oder durch ein Wasserschloss gebildet, wodruch der jeweilige Staudruck vom Atemgasstrom unabhängig wird.

Das Abschlussorgan wird bei der von Hand gesteuerten Beatmung durch den Finger des Beatmenden gebildet: Eine nicht verschlossene Ausatemöffnung entspricht der CPAP-Beatmung bei noch erhaltener Spontanatmung, periodisches Verschliessen mit dem Finger je nach Frequenz und Atemphasen der IPPV + PEEP-Beatmung bzw. der Blähdruckbeatmung.

Der Atemgasstrom hat somit gemäss der ersten Ausführungsform eine Doppelfunktion. Über ihn ist entweder der endexpiratorische Beatmungsdruck mittels dem Wasserschloss der ersten Beatmungsstufe oder der inspiratorische Beatmungsdruck mittels der zweiten Beatmungsstufe einstellbar, d.h. bei der Benutzung der zweiten Beatmungsstufe ist bei dem Beatmungsgerät gemäss der ersten Ausführungsform eine PEEP-Beatmung nicht möglich.

Gemäss einer Ausführungsform wird jedoch generell die Möglichkeit der PEEP-Beatmung dadurch erreicht, dass die Drosseleinrichtung der zweiten Beatmungsstufe ebenfalls durch ein Wasserschloss gebildet ist, das mit dem Wasserschloss der ersten Beatmungsstufe in Reihe schaltbar ist.

Diese dritte Ausführungsform ist wie bei der ersten Ausführungsform zweistufig ausgebildet, wobei jedoch alle Beatmungsstufen lediglich Wasserschlösser aufweisen, die in Reihe geschaltet sind und die additiv zueinander wirken. Die Verbindungsleitung zwischen zwei Wasserschlössern ist vorteilhaft so ausgebildet, dass keine Knickung auftreten kann. Auf diese Weise ist der inspiratorische Beatmungsdruck in allen Beatmungsstufen vom Atemgasstrom unabhängig und ausschliesslich über die Eintauchtiefe der Steigrohre in die jeweiligen Wasserpegel einstellbar, während der endexpiratorische Beatmungsdruck ausschliesslich über den Atemgasstrom steuerbar ist. Somit sind in allen Beatmungsstufen PEEP-Beatmungen möglich.

Die weitere Drosseleinrichtung gemäss der ersten Ausführungsform dient bei Verwendung lediglich zur Erzeugung des endexpiratorischen Beatmungsdrucks (PEEP/CPAP). Sie hat also nur noch die Funktion eines PEEP/CPAP-Ventils und ist vorteilhaft in der Ausatemöffnung im Patientenadapter fest integriert oder unverlierbar am Patientenadapter befestigt wie mittels eines Kettchens, da das Umstecken einer Drosseleinrichtung wie bei der ersten Ausführungsform entfällt.

Beide Wasserschlösser des zweistufigen Beatmungsgerätes sind zweckmässigerweise so ausgelegt, dass die erste Beatmungsstufe Beatmungsdrücke bis zu den in der Regel maximalen erforderlichen 3 kPa (30 cmH$_2$O) erlaubt, während die zweite Beatmungsstufe solche von 3 kPA bis max. 6 kPa (30–60 cm H$_2$O) ermöglicht.

In dem Wasserschloss der ersten Beatmungsstufe bzw. in dessen Gehäuse oder Verschlussstopfen sind vorteilhaft zwei Entlüftungsöffnungen vorgesehen, durch die das über das Steigrohr einströmende Gas abfliessen kann. Die eine Öffnung steht mit der Atmosphäre, die andere mit dem Steigrohr des Wasserschlosses der folgenden, zweiten Beatmungsstufe in Verbindung. Die zweite Beatmungsstufe ist somit nur zur ersten Beatmungsstufe zuschaltbar, wenn die mit der Atmosphäre in Verbindung stehende Öffnung verschlossen ist.

Das Wasserschloss der zweiten Beatmungsstufe, gegebenenfalls jeder folgenden Beatmungsstufe, bzw. das Gehäuse oder der Verschlussstopfen weist vorteilhaft drei Öffnungen auf. Die eine Öffnung steht über eine Hülse mit einem verschiebbaren am oberen Ende verschlossenen Steigrohr über eine Bohrung im Steigrohr innerhalb des Hülsenbereichs in Verbindung. Die Hülsenlänge zwischen den Abdichtungen zur Hindurchführung des Steigrohrs entspricht mindestens dem Einstellbereich des Steigrohrs bzw. dem Verschiebungsbereich. Die beiden anderen Öffnungen dienen zur Entlüftung. Durch sie kann das über das Steigrohr einströmende Gas vollständig abströmen. Die eine Entlüftungsöffnung steht dabei mit der Atmosphäre in Verbindung. Über die andere kann bei Bedarf ein weiteres Wasserschloss einer weiteren Beatmungsstufe in Reihe geschaltet werden, wobei dieses weitere Wasserschloss im Aufbau dem Wasserschloss der zweiten Beatmungsstufe entspricht. Die Zuschaltung jedes Wasserschlosses einer weiteren Beatmungsstufe erfolgt in gleicher Weise wie die Zuschaltung des Wasserschlosses der zweiten Beatmungsstufe zu dem der ersten Beatmungsstufe über eine Verschlusseinrichtung der zur Atmosphäre führenden Entlüftungsöffnung.

Zweckmässigerweise ist die Verbindungsleitung zwischen zwei aufeinanderfolgenden Wasserschlössern starr ausgebildet und vorteilhaft in der Gehäuseverbindung der Wasserschlösser integriert. Zwar ist eine Schlauchverbindung möglich, jedoch muss dann darauf geachtet werden, dass ein Abknicken dieser Schlauchverbindung oder das Entstehen eines Wassersackes in der Schlauchverbindung verhindert ist, da dies zu einem den Patienten gefährdenden Druck führen kann.

Wie erwähnt, ist die jeweils folgende Beatmungsstufe erst dann zuschaltbar, wenn eine zur Atmosphäre führende Entlüftungsöffnung verschlossen ist. Zweckmässigerweise ist das Verschlussglied hierzu durch einen Stöpsel gebildet, der am Steigrohr oder dem an diesem abgebrachten T-Verbindungsstück unverlierbar wie mittels eines Kettchens derart befestigt ist, dass das Verschlussglied nur bei vollständig in den Wasserpegel eingetauchtem Steigrohr (entsprechend 3 kPa in der ersten Beatmungsstufe) in die mit der Atmosphäre in Verbindung stehenden Entlüftungsöffnung des Wasserschlosses der jeweiligen Beatmungsstufe zum Zuschalten des Wasserschlosses der folgenden Beatmungsstufe gesteckt werden kann. Bei inspiratorischen Beatmungsdrücken unter dem Maximalwert der jeweiligen Beatmungsstufe, 3 kPa in der ersten Beatmungsstufe, gibt das Verschlussglied automatisch die Entlüftungsöffnung frei, da es mit dem Steigrohr aus der Entlüftungsöffnung herausgezogen wird. Das heisst, bei Drücken unterhalb dieses Grenzdruckes ist die jeweils folgende Beatmungsstufe automatisch abgetrennt. Nachdem die Reanimation von Neugeborenen aufgrund allgemeiner Empfehlung stets mit inspiratorischen Beatmungsdrücken von maximal 2 kPa begonnen werden soll, kann so sichergestellt werden, dass die zweite Beatmungsstufe nicht versehentlich zugeschaltet wird oder bleibt.

Zweckmässig ist das Verschlussglied auch so ausgebildet, dass dann, wenn beim Abwärtsschieben des Steigrohres das Verschlussglied unbeabsichtigt in die Entlüftungsöffnung hineinrutschen sollte, bereits ein Staudruck von unter 0,5 kPa ausreicht, das Verschlussglied aus der Entlüftungsöffnung herauszudrücken.

Dadurch ist es auch möglich, das Steigrohr des Wasserschlosses der folgenden Beatmungsstufe, wie das der zweiten Beatmungsstufe, so zu skalieren, dass es mit dem Enddruck der jeweils vorhergehenden Beatmungsstufe beginnt, da die folgende Stufe nur dann zur vorhergehenden zugeschaltet werden kann, wenn die vorhergehende ihren maximalen Druckwert, bei der ersten Beatmungsstufe 3 kPa, erreicht hat.

Zur Vermeidung einer Verwechslung skalierter Steigrohre können deren Durchmesser unterschiedlich sein.

Die für das Wasserschloss der zweiten und folgenden Stufe verwendete Ausführungsform mit Hülse kann auch bei dem Wasserschloss der ersten Beatmungsstufe verwendet werden. Der Atemgas zuführende Schlauch sowie der zum Patientenadapter führende Schlauch sind dann jeweils starr am Gehäuse des Wasserschlosses der ersten Beatmungsstufe angeschlossen, statt an dem Steigrohr. Bei einer derartigen Ausbildung müssen die Schläuche bei der Einstellung des Druckes durch Verschieben des Steigrohres nicht mitbewegt werden.

Bei der automatisch gesteuerten Ausführung besteht das Abschlussorgan vorteilhaft aus einem zylindrischen Rohr und einer darin drehbaren Kugel mit zur Rohrachse senkrechten Drehachse, wobei die Kugel auf einer zur Drehachse parallelen Seite abgeplattet ist und die Querschnitte von Rohr und Kugel einander angepasst sind. Angetrieben wird die Kugel von einem druckluft- oder batteriebetriebenen Elektromotor mit regelbarer Drehzahl, wobei pro Umdrehung zwei Atemzyklen durchlaufen werden. Die Atemphasen sind nicht einstellbar, sondern von der jeweiligen Kugelabplattung abhängig.

Die mit der Erfindung erzielten Vorteile bestehen neben den bekannten und mittlerweile wieder geschätzten Vorteilen einer vereinfachten und überschaubaren Gerätebauweise insbesondere darin, dass die von Hand gesteuerte Ausführungsform störungsfrei arbeitet und keiner Wartung bedarf, da sie keine sich bewegenden und daher keine dem Verschleiss unterliegenden oder zum Verkleben neigenden Teile oder Ventile aufweist, dass Patient und Beatmungsgerät, die Handlich- und Übersichtlichkeit fördernd nur mit einem einzigen Atemschlauch verbunden werden müssen, dass die Beatmung Neugeborener risikoarm in zwei Stufen erfolgt, wobei der inspiratorische Beatmungsdruck am Gerät voreingestellt und bei Bedarf sofort geändert werden kann und der Beatmende die Beatmungsart, -frequenz und -phasen direkt am Patienten durch entsprechendes Abschliessen der Ausatemöffnung im Patientenadapter unmittelbar mit seinem Finger steuern kann, ohne sich vom Patienten abwenden zu müssen. Die sonst notwendigen komplizierten Einstellungen am Gerät mit einer Vielzahl von versehentlich verstellbaren Knöpfen und Schaltern entfallen dadurch ebenso, wie das damit verbundene Risiko von Fehlbedienungen.

Die zweistufige Anordnung des Beatmungsvorganges ist besonders vorteilhaft. Langjährige klinische Erfahrung bei der Reanimation Neugeborener hat nämlich gezeigt, dass die Beatmungsdrücke prinzipiell variabel sein müssen, vor allem aber Drücke von über 3 kPa (30 cm $H_2O$) bei Neugeborenen gehäuft zu Komplikationen in Form von Lungenrissen führen und nur in Ausnahmefällen und zwar ausschliesslich zur initialen Entfaltung der Lunge therapeutisch notwendig werden, im Normalfall aber solche bis zu 3 kPa (30 cm $H_2O$) sowohl zur initialen Entfaltung der Lunge als auch zur künstlichen Unterstützung der Atmung ausreichen.

Die Erfindung berücksichtigt erstmals diese bedeutende in der Beatmungstechnik Neugeborener zwar bekannte, aber in keinem bekannten Beatmungsgerät verwirklichte klinische Erkenntnis und unterteilt den Beatmungsvorgang entsprechend in zwei Stufen, wobei der kritische Beatmungsdruck von 3 kPa (30 cm $H_2O$) entsprechend der Wirkung eines Sicherheitsventils, die Grenze zwischen erster und zweiter Stufe bildet. Das bisher meist benützte einfache Beatmungsgerät, nämlich der Atembeutel, arbeitet mit praktisch fest vorgegebenen, überdies unkontrollierbaren Drücken. Nur bei den teuren Beatmungsgeräten ist der inspiratorische Beatmungsdruck, allerdings ohne jegliche Sperre, zwischen minimalem und maximalem Druck kontinuierlich einstellbar. Beides ist, wie erwähnt, gefährlich. Bei der Erfindung sind die höheren, gefährlichen Beatmungsdrücke von 3–7 kPa (30–70 cm $H_2O$) normalerweise blockiert und nur über zwei vorzunehmende Handgriffe, was einen zusätzlichen Sicherheitsfaktor darstellt, verfügbar und damit vor unbeabsichtigter Benützung geschützt.

Der erste Handgriff besteht bei der ersten Ausführungsform im Umstecken der Drosseleinrichtung vom Patientenadapter auf die Entlüftungsöffnung des Wasserschlosses, was aber allein noch keine Druckerhöhung ermöglicht. Erst zusammen mit dem zweiten vorzunehmenden Handgriff wird es mittels der zweiten Beatmungsstufe möglich, über höhere Atemgasströme zu höheren Drücken zu gelangen. Dazu werden je nach erforderlichem Blähdruck Atemgasströme bis zu $12 \cdot 10^{-3}$ m³/min notwendig, während in der ersten Beatmungsstufe nur solche zwischen $2 \cdot 10^{-3}$ und $5 \cdot 10^{-3}$ m³/min auftreten und vom einzustellenden PEEP bzw. CPAP abhängen. Nach erfolgter Entfaltung der Lunge, erkennbar am sich hebenden Brustkorb, sichert allein das Abnehmen der Drosseleinrichtung von der Entlüftungsöffnung des Wasserschlosses, dass der kritische Beatmungsdruck von 3 kPa (30 cm $H_2O$) nicht mehr überschritten wird und daher gefahrlos weiterbeatmet werden kann. Allerdings muss bei Benützung der zweiten Beatmungsstufe bei der ersten Ausführungsform auf PEEP-Beatmung verzichtet werden. Da aber damit nur die initiale Blähdruckbeatmung zur Entfaltung der Lunge durchgeführt werden soll, wozu nur inspiratorische aber keine endexspiratorischen Blähdrücke der Lunge notwendig sind, stellt dies mitunter keinen Nachteil dar.

Neben den vorstehend erwähnten Vorteilen bestehen die bei der zweiten Ausführungsform erreichten Vorteile neben den voneinander getrennt einstellbaren Beatmungsdrücken während In- und Expiration (Einatmung bzw. Ausatmung) insbesondere darin, dass in beiden Beatmungsstufen PEEP-Beatmungen möglich sind. Weiter besitzt die (weitere) Drosseleinrichtung nur noch die Funktion eines PEEP/CPAP-Ventils und kann am Patientenadapter unverlierbar befestigt werden. Schliesslich ist das Beatmungsgerät noch handlicher und bedienungsfreundlicher und auch bedienungssicherer, ohne dass dadurch die Herstellung oder die Wartung aufwendiger wäre. Insbesondere führt ein falscher Zusammenbau oder ein Abknicken der Atemschläuche zu keiner Gefährdung des Patienten.

Schliesslich kann das Beatmungsgerät gemäss der zweiten und auch der dritten Ausführungsform ausserdem als zwei- oder mehrstufiges Wasservakuummeter und -regler für die Thoraxdrainage eingesetzt werden. Die Serienschaltung mehrerer Wasserschlösser erfolgt in der vorstehend erläuterten Weise, wobei jedoch die mit der Atmosphäre in Verbindung stehenden Entlüftungsöffnungen vom Wasserschloss der ersten Beatmungsstufe, die nun die zweite bzw. letzte Saugstufe bildet, nicht in dessen Gehäuse sondern als Belüftungsöffnung in dem Wasserschloss der zweiten Beatmungsstufe, die nun die erste Saugstufe bildet, angeordnet ist, wobei analoges bei einer Reihenschaltung mehrerer Wasserschlösser gilt. Weiter ist das am Steigrohr der ersten Beatmungsstufe angebrachte Verschlussglied für die Entlüftungsöffnung nunmehr am Steigrohr der ersten Saugstufe befestigt. Schliesslich sind auch die Skalen der Steigrohre entsprechend zu ändern, derart, dass das Steigrohr der ersten Saugstufe nunmehr die Skale des Steigrohrs der ersten Beatmungsstufe enthält usw. Weiter sind die Vakuumquelle und der Patient an der mit der Atmosphäre in Verbindung stehenden als Ansaugöffnung dienenden Öffnung der ersten Saugstufe anzuschliessen. Bei einer mehrstufigen Ausbildung gilt Sinngemässes.

Weitere Merkmale der vorliegenden Erfindung ergeben sich aus nachfolgender Beschreibung eines Ausführungsbeispiels, das an Hand schematischer Zeichnungen näher erläutert wird. Es zeigen

Fig. 1 eine bevorzugte Ausführungsform der Erfindung;

Fig. 2 vergrössert ein Steuerventil;

Fig. 3 und 4 die Beatmung eines Patienten mit der in Fig. 1 dargestellten Ausführungsform der Erfindung unter Verwendung nur der ersten (Fig. 3) bzw. zusätzlich der zweiten (Fig. 4) Beatmungsstufe, wobei Pfeile innerhalb der Atemgasleitungen die Richtung des Atemgasstromes während der Inspiration (Fig. a) bzw. der Exspiration (Fig. b) angeben.

Fig. 5 eine zweite wesentliche Ausführungsform der Erfindung;

Fig. 6 das Beatmungsgerät bei der Anwendung bei der Thoraxdrainage.

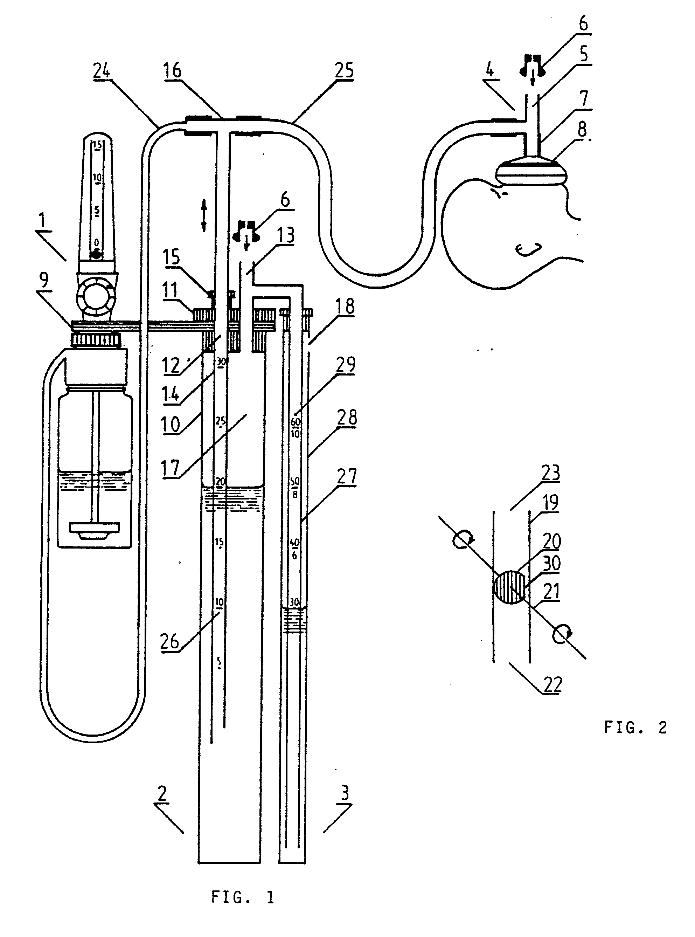

Wie Fig. 1 zeigt, besteht das Beatmungsgerät im wesentlichen aus einem üblichen Durchflussmengenregler 1 für Atemgas mit Anzeige des Atemgasstromes in l/min ($10^{-3}$ m³/min) einer ersten Beatmungsstufe mit einem Wasserschloss 2, einer zweiten Beatmungsstufe mit einem Flüssigkeitsmanometer 3 als Druckbegrenzer und einem Patientenadapter 4 mit einer auf dessen Ausatemöffnung 5 aufsteckbaren Drossel 6 als Drosseleinrichtung und einem Steckanschluss 7 für eine Gesichtsmaske 8 bzw. für einen (nicht dargestellten) Trachealtubus. Der Durchflussmengenregler 1 und das Wasserschloss 2 sind durch einen Adapter 9 miteinander starr verbunden.

Das Wasserschloss 2 besteht aus einem bis zur Einfüllmarke mit entkeimtem Wasser gefüllten Wassergefäss 10 aus z.B. Acrylglas, das durch einen Verschlussstopfen 11 mit Öffnungen 12 und 13 abgedichtet ist. Die Öffnung 12 dient zur Aufnahme eines skalierten Steigrohrs 14 aus z.B. Acrylglas, das mittels einer Feststellschraube 15 in einer gewünschten Eintauchtiefe fixiert werden kann. Über die Öffnung 13 wird das Wassergefäss 10 entlüftet. Das Steigrohr 14 ist mittels einem in das Steigrohr 14 starr integrierten T-Verbindungsstück 16 zum einen durch einen Atemschlauch 24 über den Durchflussmengenregler 1 mit einer (nicht dargestellten) Druckgasquelle für Atemgas und zum anderen durch einen Atemschlauch 25 über den Patientenadapter 4 mit dem Patienten verbunden.

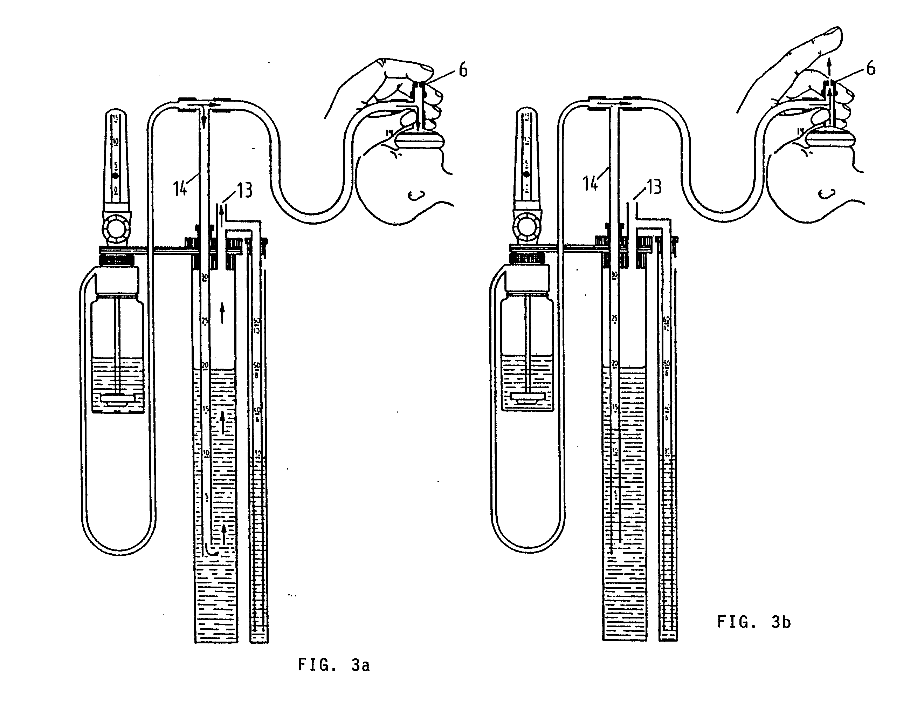

Der inspiratorische Beatmungsdruck wird in dieser erläuterten ersten Beatmungsstufe, in der sich die freien Querschnitte von Entlüftungsöffnung 13 und Steigrohr 14 in etwa entsprechen, allein von der voreingestellten Eintauchtiefe (maximal z.B. 30 cm ≙ Beatmungsdruck von 3 kPa (30 cm $H_2O$)) des skalierten Steigrohrs 14 in das Wasserniveau begrenzt (Fig. 3a). Wenn die gemäss dem am Steigrohr 14 vorgegebenen Druck mögliche Blähung der Lunge erreicht ist, entweicht das einströmende Atemgas vollständig über das Wasserschloss 2 durch die Öffnung 13. Der endexspiratorische Beatmungsdruck (PEEP bzw. CPAP) ist bei auf die Ausatemöffnung 5 aufgesteckter Drossel 6 allein vom Atemgasstrom durch die Drossel 6 abhängig (Fig. 3b). Der sich so vor der Drossel 6 und damit auch in den Luftwegen des Patienten ausbildende Ist-Staudruck ist an der Skala 26 am Steigrohr 14 ablesbar.

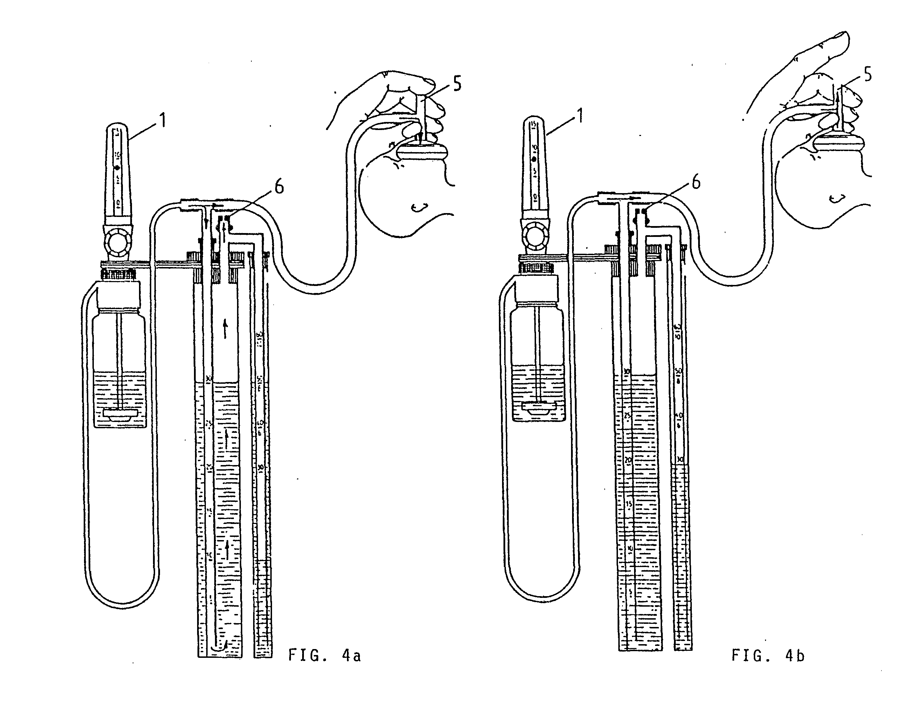

Die zweite Beatmungsstufe erweitert die erste und unterscheidet sich von ihr durch die dann auf die Entlüftungsöffnung 13 aufgesteckte Drossel 6 als Drosseleinrichtung (Fig. 4a, 4b), wodurch sich im Raum 17 vor der Drossel 6 ein zusätzlicher Staudruck ausbildet, der allein vom Atemgasstrom durch die Drossel 6 abhängt und von dem Flüssigkeitsmanometer 3 sowohl angezeigt, als auch auf 4 kPa (40 cm $H_2O$) begrenzt wird. Dieser dynamisch erzeugte Staudruck addiert sich zu dem am Steigrohr 14 eingestellten hydrostatischen. Auf diese Weise werden mit der zweiten Beatmungsstufe Beatmungsdrücke von 3 kPa–7 kPa (30–70 cm $H_2O$) möglich, maximale Eintauchtiefe des Steigrohrs 14 im Wassergefäss 10 des Wasserschlosses 2 vorausgesetzt.

Das Flüssigkeitsmanometer 3 besteht aus zwei konzentrisch ineinander steckenden Acrylglas-Rohren 27, 28, die nach Art eines U-Rohrs hintereinander geschaltet sind. Das innere Rohr 27 steht mit Raum 17, das äussere Rohr 28 über eine Bohrung 18 mit der Atmosphäre in Verbindung. Bei Überdruck im Raum 17 sinkt die innere Flüssigkeitssäule ab, während die äussere entsprechend ansteigt. Der dazugehörige Beatmungsdruck ist in cm $H_2O$ (0,1 kPa) an der Skala 29 am äusseren Rohr 28 ablesbar, wobei der Skalenbeginn (= Füllmarke) auf die maximale Eintauchtiefe des Steigrohrs 14 des Wasserschlosses 2 von hier 30 cm kalibirert ist. Unter dem jeweiligen Beatmungsdruck in cm $H_2O$ sind auf der Skala 29 noch der dazu notwendige Atemgasstrom in l/min ($10^{-3}$ m³/min) angegeben. Durch Einstellen des entsprechenden Wertes am Durchflussmengenregler 1 ist auch in der zweiten Beatmungsstufe der Beatmungsdruck voreinstellbar. Als Flüssigkeitsfüllung des Manometers kann die unter der Handelsbezeichnung Chlorhexamed, ein Mund- und Rachen-Antiseptikum, erhältliche Flüssigkeit verwendet werden, da sie zum einen desinfizierend wirkt und zum anderen durch ihre rötliche Farbe den Flüssigkeitspegel gut erkennen lässt.

Fig. 2 zeigt ein Steuerventil für ein Ausführungsbeispiel mit automatischer Steuerung. Es besteht aus einem zylindrischen Rohr 19 und einer darin rotierenden Kugel 20 mit zur Rohrachse senkrechten Drehachse 21, wobei die Kugel 20 auf einer zur Drehachse 21 parallelen Seite, den Atemphasen entsprechend, eine Abplattung 30 besitzt. Angetrieben wird die Kugel von einem (nicht dargestellten) regelbaren Motor. Mit einer Öffnung 22 wird das Steuerventil auf die Ausatemöffnung 5 des Patientenadapters 4 aufgesteckt, wobei auf eine Öffnung 23 bei Bedarf die Drossel 6 aufgesteckt werden kann.

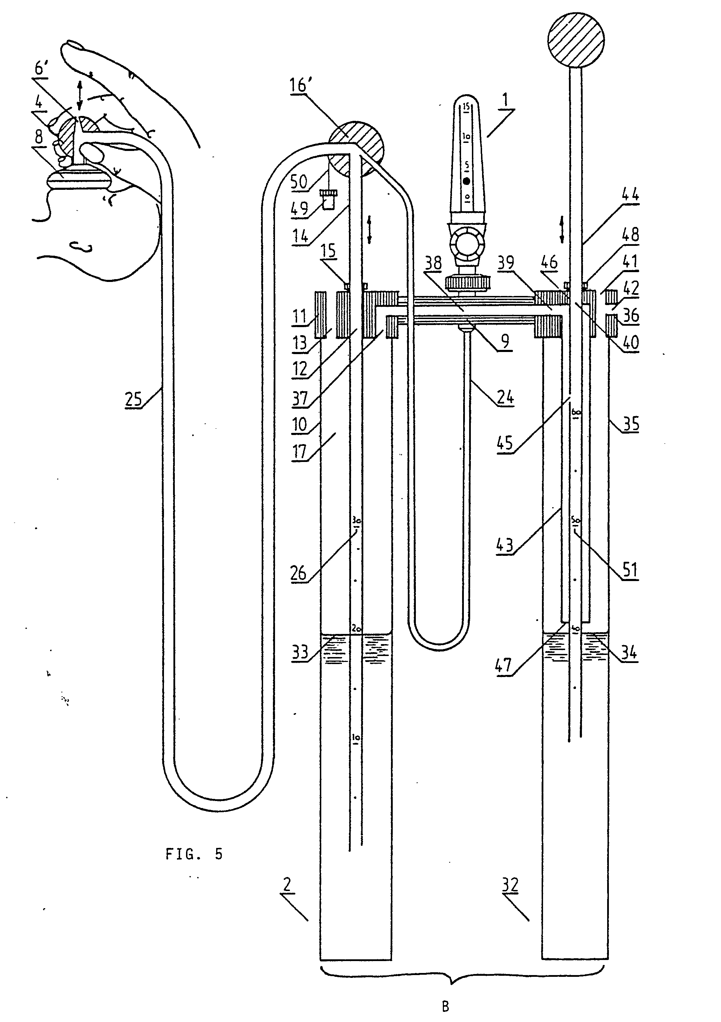

Das in Fig. 5 dargestellte Beatmungsgerät B zeigt die zweite wesentliche Ausführungsform. Es sind bei der Erläuterung dieser Ausführungsform die gleichen Bezugszeichen wie bei der Erläuterung der ersten Ausführungsform (Fig. 1–4) verwendet, wenn gleiche Bauelemente betroffen sind.

Das erfindungsgemässe Beatmungsgerät B gemäss Fig. 5 ist bei der dargestellten Ausführungsform zweistufig verwendbar. Die Grundelemente jeder Beatmungsstufe sind durch Wasserschlösser gebildet.

Das Beatmungsgerät B besteht im wesentlichen aus einem Durchlussmengenregler 1 für Atemgas mit Anzeige des Atemgasstromes in l/min ($10^{-3}$ m³/min), einem Wasserschloss 2 einer ersten Beatmungsstufe, einem zuschaltbaren Wasserschloss 32 einer zweiten Beatmungsstufe und einem Patientenadapter 4 mit einer in dessen Ausatemöffnung angeordneten PEEP/CPAP-Drossel 6' als Drosseleinrichtung und einem Anschluss für eine Gesichtsmaske 8 bzw. für einen (nicht dargestellten) Trachealtubus. Der Durchflussmengenregler 1 und die Wasserschlösser 2, 32 der verschiedenen Beatmungsstufen sind durch einen Adapter oder eine Befestigung 9 miteinander starr verbindbar.

Die Wasserschlösser 2, 32 der beiden Beatmungsstufen bestehen aus im wesentlichen identischen bis zu einer jeweiligen Einfüllmarkierung 33 bzw. 34 mit entkeimtem Wasser gefüllten Wasserbehältern 10 bzw. 35 aus beispielsweise Acrylglas, in die ein Verschlussstopfen 11 bzw. 36 nach aussen dicht eingesetzt, beispielsweise eingeschraubt, ist. Die Verschlussstopfen 11, 36 sind zweckmässigerweise mit der Befestigung 9 fest verbunden, gegebenenfalls mit dieser einstückig ausgebildet.

Der Verschlussstopfen 11 des Wasserschlosses 2 der ersten Beatmungsstufe besitzt Öffnungen 12, 13, 37. Die Öffnung 12 dient zur dichten Durchführung eines skalierten Steigrohrs 14 aus beispielsweise Acrylglas, das mittels einer Feststellschraube 15 in einer gewünschten Eintauchtiefe fixiert werden kann. Die Öffnung 13 steht mit Atmosphäre in Verbindung. Der Raum 17 des Wasserschlosses 2 über der Markierung 33 wird über diese Öffnung 13 entlüftet. Über die Öffnung 37 steht der Raum 17 mittels einer Verbindungsleitung 38 mit dem Wasserschloss 32 der zweiten Beatmungsstufe in Verbindung.

Das Steigrohr 14 ist mittels einem im Steigrohr 14 starr integrierten T-Verbindungsstück 16' zum einen durch einen Atemschlauch 24 über den Durchflussmengenregler 1 mit einer (nicht dargestellten) Druckgasquelle für Atemgas und zum anderen über einen Atemschlauch 25 mit dem Patientenadapter 4 und über diesen mit dem Patienten verbunden.

Der Verschlussstopfen 36 des Wasserschlosses 32 der zweiten Beatmungsstufe besitzt Öffnungen 39, 40, 41, 42. Die Öffnung 39 führt in eine Hülse 43, in der das Steigrohr 44, das am oberen Ende verschlossen ist, nach aussen dicht verschiebbar ist. Die Hülse 43 muss nicht transparent sein und kann beispielsweise aus einem Messingrohr oder dergleichen bestehen. Innerhalb der Hülse 43 weist das verschiebbare Steigrohr 44 eine Öffnung 45 auf, über die das Steigrohrinnere somit mit dem Raum 17 des Wasserschlosses 2 der ersten Beatmungsstufe in Verbindung steht. Für die Länge der Hülse 43 gilt, dass der Einstellbereich, d.h., der Verschiebebereich des Steigrohrs 44 in cm Wassersäule (entsprechend 0,1 kPa) der Hülsenlänge in cm zwischen Dichtungen 46 und 47 entspricht. Die Öffnung 40 dient, zusammen mit der Dichtung 46 und der Dichtung 47, zur nach aussen dichten Hindurchführung des Steigrohrs 44. Es kann ebenfalls eine Feststellschraube 48 vorgesehen sein. Die Öffnungen 41 und 42 dienen zur Entlüftung. Durch sie kann das über das Steigrohr 44 in das zweite Wasserschloss 32 einströmende Gas vollständig abströmen. Über die Öffnung 42 kann gegebenenfalls ein weiteres Wasserschloss entsprechend einer weiteren Beatmungsstufe in Reihe zugeschaltet werden.

Zur Vermeidung einer unbeabsichtigten Knickung der Verbindungsleitung 38 zwischen den Wasserschlössern 2 und 32 ist diese starr ausgebildet und bei dem dargestellten Ausführungsbeispiel in der Befestigung 30 der beiden Wasserschlösser 2, 32 bzw. deren Verschlussstopfen 11, 36 integriert.

Es sei erwähnt, dass das Wasserschloss 2 der ersten Beatmungsstufe die gleiche Ausbildung wie das Wasserschloss 32 der zweiten Stufe, also mit Zufuhr über eine das Steigrohr umgebende Hülse, aufweisen kann. In einem solchen Fall ist das T-Verbindungsstück 16′ starr an einer Zuführöffnung im Verschlussstopfen 11 angebracht und braucht bei einer Verschiebung des Steigrohrs 14 nicht mehr mitbewegt werden.

Das Zuschalten des Wasserschlosses 32 der zweiten Beatmungsstufe zum Wasserschloss 2 der ersten Beatmungsstufe erfolgt durch Verschliessen der Entlüftungsöffnung 13 im Verschlussstopfen 11 des Wasserschlosses 2 der ersten Beatmungsstufe. Als Verschlussglied dazu eignet sich insbesondere ein Stöpsel 49. Um zu verhindern, dass der Stöpsel 49 in die Entlüftungsöffnung 13 eingesetzt werden kann, bevor der mit dem Wasserschloss 2 der ersten Beatmungsstufe erreichbare maximale Druck tatsächlich erreicht ist, ist der Stöpsel 49 unverlierbar, beispielsweise mittels eines Kettchens 50, am T-Verbindungsstück 16′ oder direkt am Steigrohr 14 so befestigt, dass der Stöpsel 49 nur bei ganz eingetauchtem Steigrohr 14 in die Öffnung 13 eingeführt werden kann. Dies erfolgt durch geeignete Bemessung des Kettchens 50.

In einem solchen Fall kann auch das Steigrohr 44 des folgenden Wasserschlosses 32 eine Skala 51 aufweisen, die mit dem mit dem Wasserschloss 2 der ersten Beatmungsstufe erreichbaren maximalen Druckwert beginnt (beispielsweise 3 kPa). Zur Vermeidung von Verwechslungen bei skalierten Steigrohren sind diese zweckmässig so ausgebildet, dass sie jeweils nur in dem Wasserschloss der zugeordneten Stufe verwendbar sind. Zweckmässig sind insbesondere unterschiedliche Querschnitte.

Die Entlüftungsöffnungen in den Verschlussstopfen können wie bei dem Verschlussstopfen 11 getrennt oder wie bei dem Verschlussstopfen 36 gemeinsam als (z.B.) T-förmige Durchführung ausgebildet sein. Es sollte lediglich sichergestellt sein, dass nicht versehentlich die Entlüftung des letzten Wasserschlosses der in Betrieb befindlichen Beatmungsstufen verhindert werden kann.

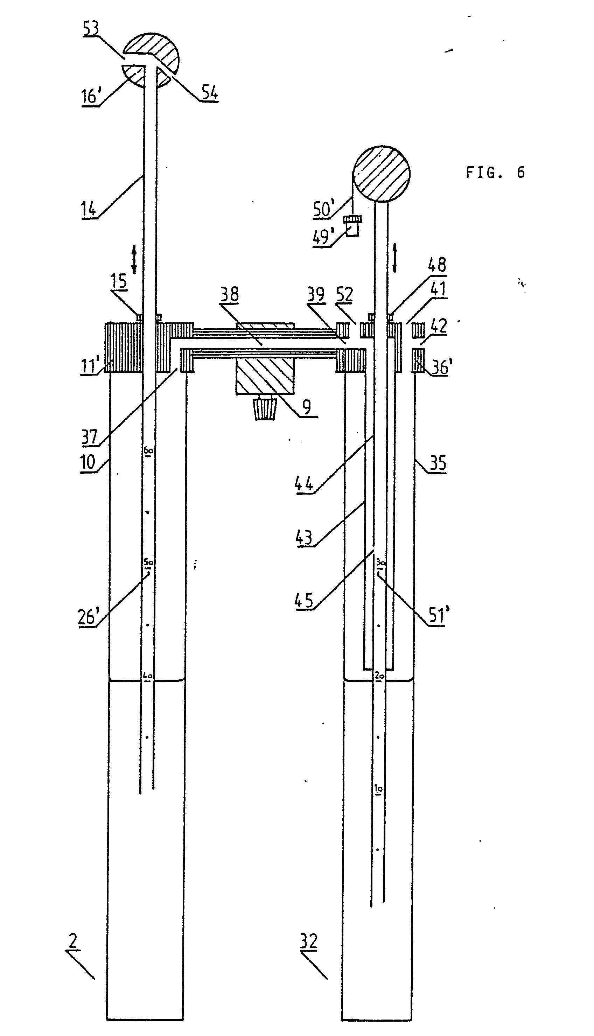

Fig. 6 zeigt im wesentlichen ein Gerät mit der gleichen Ausführung wie in Fig. 5, wobei jedoch das Gerät gemäss Fig. 6 als Wasservakuumregler und -meter zur Thoraxdrainage ausgebildet ist.

Bei diesem Gerät bildet das Wasserschloss 32 die erste Saugstufe und bildet das Wasserschloss 2 die zweite Saugstufe. Mit den Entlüftungsöffnungen 41, 42 des Wasserschlosses 32 sind nun eine Vakuumquelle bzw. eine zum Patienten führende Leitung angeschlossen. An dem Steigrohr 44 des Wasserschlosses 32 ist ein Stöpsel 49′ mittels eines Kettchens 50′ befestigt. In dem Verschlussstopfen 36′ befindet sich zusätzlich eine Öffnung 52, die mittels des Stöpsels 49′ bei in das Wasserschloss bzw. dessen Gehäuse 35 eingeschobenem Steigrohr 44 verschliessbar ist, wodurch zur Bildung der zweiten Saugstufe das Wasserschloss 2 zugeschaltet wird. Das Wasserschloss 2 weist an seinem T-Verbindungsstück 16′ keine Anschlussleitungen 24 oder 25 mehr auf, sondern erlaubt eine Verbindung zur Umgebungsluft über die freigegebenen Öffnungen 53, 54, in denen die Verbindungsschläuche 24 und 25 gemäss Fig. 1 eingesetzt waren. Weiter ist die Zugangsöffnung 13 in dem Verschlussstopfen 11′ nicht vorgesehen. Weiter ist festzustellen, dass die Steigrohre 44 und 14 Skalen 51′ bzw. 26′ aufweisen, die sich von den Skalen 51 bzw. 26 gemäss Fig. 5 unterscheiden, insbesondere gegeneinander ausgetauschte Skalenwerte besitzen.

Die an einer der Öffnungen 41, 42 angeschlossene Vekauumquelle erzeugt im Patienten einen Unterdruck, der durch Verschieben des Steigrohrs 44 im Wasserschloss 32 in der ersten Saugstufe auf einen an der Skala 51′ ablesbaren Wert begrenzt ist. Wenn bei dem durch die Begrenzung durch das Wasserschloss 32 erreichbaren grössten Unterdruck, d.h. bei vollständig in das Wasserschloss 32 eingeschobenem Steigrohr 44 ein noch höheres Vakuum erforderlich ist, wird der Stöpsel 49′ in die Öffnung 52 eingesetzt, wodurch das Wasserschloss 2 der zweiten Saugstufe zugeschaltet wird.

Es sei erwähnt, dass einerseits auch hier das Wasserschloss 2 eine mit der Ausbildung des Wasserschlosses 32 vergleichbare Ausbildung besitzen kann (Hülse im Verschlussstopfen, in ihr nach aussen dicht verschiebliches Steigrohr und Öffnung im Steigrohr innerhalb der Hülse). Weiter kann der Verschlussstopfen 36′ des Wasserschlosses 32 statt der Öffnungen 41, 42 lediglich eine davon aufweisen, wobei in diese dann ein T-Verbindungsstück eingesetzt ist, mittels dem die Verbindung zwischen Vakuumquelle und Patient erreichbar ist, ähnlich einer Ausführungsform, die bei der Erläuterung des Beatmungsgerätes B gemäss Fig. 5 erläutert worden ist. Schliesslich kann auch das Gerät gemäss Fig. 6 mehrstufig ausgebildet sein, beispielsweise dadurch, dass mit den Öffnungen 41 und 42 über eine der Verbindungsleitung 38 entsprechende Verbindungsleitung ein weiteres Wasserschloss, das dem Wasserschloss 32 in der Ausbildung entspricht, angeschlossen ist, wobei dieses Wasserschloss nun die erste Saugstufe bildet, usw. Bei einer Ausbildung aller Wasserschlösser wie das Wasserschloss 32 ist die Hinzufügung weiterer Wasserschlösser an beiden Seiten möglich.

Auch hier ist lediglich sicherzustellen, dass in dem letzten Wasserschloss der in Betrieb befindlichen Saugstufe ein auch nur versehentliches Verschliessen der mit der Umgebungsluft in Verbindung stehenden Öffnung (hier die Öffnungen 53 und 54) verhindert wird.

Es sei erwähnt, dass selbstverständlich die Ausbildung der Verschlussstopfen so sein kann, dass sie für beide Betriebsarten geeignet ist. In einem solchen Fall sind betriebsartabhängig entweder die Öffnung 52 bei Verwendung als Beatmungsgerät gemäss Fig. 5 oder die Öffnung 13 bei Verwendung zur Thoraxdrainage durch geeignete Mittel zu verschliessen.

**Patentansprüche**

1. Beatmungsgerät zur Reanimation von Neugeborenen, mit einem Wasserschloss (2) als Einatemventil mit einem mit dem Steigrohr (14) des Wasserschlosses (2) verbundenen T-Verbindungsstück (16), durch das das Wasserschloss (2) über Atemschläuche (24, 25) mit einer Atemgasquelle und mit einem Patientenadapter (4) verbunden ist, der eine mit der Atmosphäre in Verbindung stehende verschliessbare freie Öffnung (5) für die Ausatmung besitzt, bei dem die Menge des von der Atemgasquelle zugeführten Atemgases durch einen Regler (1) einstellbar ist, bei dem die maximale Eintauchtiefe des Steigrohrs (14) den maximalen inspiratorischen Beatmungsdruck bestimmt und bei dem die Verschlussdauer der Ausatemöffnung (5) Atemfrequenz und Atemphase bestimmt, dadurch gekennzeichnet, dass zwei Beatmungsstufen vorgesehen sind, deren erste Beatmungsstufe durch das Wasserschloss (2) gebildet ist, und deren zweite Beatmungsstufe zuschaltbar ist und durch eine in die Entlüftungsöffnung (13) des Wasserschlosses (2) der ersten Beatmungsstufe angeordnete Drosseleinrichtung (6) gebildet ist, wobei sowohl der Querschnitt der Drosseleinrichtung (6) als auch die Menge des von der Atemgasquelle zugeführten Atemgases den Wert des inspiratorischen Beatmungsdruckes bestimmen.

2. Beatmungsgerät nach Anspruch 1, gekennzeichnet durch ein Sicherheitsventilglied (3) in der zweiten Beatmungsstufe, durch das der inspiratorische Beatmungsdruck auf einen maximalen Wert begrenzbar ist.

3. Beatmungsgerät nach Anspruch 2, dadurch gekennzeichnet, dass das Sicherheitsventilglied durch ein Flüssigkeitsmanometer (3) nach Art eines U-Rohres gebildet ist.

4. Beatmungsgerät zur Reanimation von Neugeborenen, mit einem Wasserschloss (2) als Einatemventil mit einem mit dem Steigrohr (14) des Wasserschlosses (2) verbundenen T-Verbindungsstück (16), durch das das Wasserschloss (2) über Atemschläuche (24, 25) mit einer Atemgasquelle und mit einem Patientenadapter (4) verbunden ist, der eine mit der Atmosphäre in Verbindung stehende verschliessbare freie Öffnung (5) für die Ausatmung besitzt, bei dem die Menge des von der Atemgasquelle zugeführten Atemgases durch einen Regler (1) einstellbar ist, bei dem die maximale Eintauchtiefe des Steigrohrs (14) den maximalen inspiratorischen Beatmungsdruck bestimmt, und bei dem die Verschlussdauer der Ausatemöffnung (5) Atemfrequenz und Atemphase bestimmt, dadurch gekennzeichnet, dass in der Ausatemöffnung (5, 23) des Patientenadapters (4) eine Drosseleinrichtung (6, 6') zur Erzeugung des endexpiratorischen Beatmungsdrucks zuschaltbar ist, wobei sowohl der Querschnitt der zuschaltbaren Drosseleinrichtung (6, 6'), als auch die Menge des von der Atemgasquelle zugeführten Atemgases den Wert des endexpiratorischen Beatmungsdrucks bestimmen.

5. Beatmungsgerät nach einem der Ansprüche 1 bis 4, gekennzeichnet durch eine einzige umsteckbare Drosseleinrichtung (6).

6. Beatmungsgerät nach einem der Ansprüche 1 bis 4, dadurch gekennzeichnet, dass als zuschaltbare Drosseleinrichtung (6) eine im Querschnitt verstellbare Drosseleinrichtung (6) angeordnet ist, wobei der Wert der entsprechenden Beatmungsdrücke von der Menge des von der Atemgasquelle zugeführten Atemgases unabhängig wird.

7. Beatmungsgerät zur Reanimation von Neugeborenen, mit einem Wasserschloss (2) als Einatemventil mit einem mit dem Steigrohr (14) des Wasserschlosses (2) verbundenen T-Verbindungsstück (16'), durch das das Wasserschloss (2) über Atemschläuche (24, 25) mit einer Atemgasquelle und mit einem Patientenadapter (4) verbunden ist, der eine mit der Atmosphäre in Verbindung stehende verschliessbare freie Öffnung für die Ausatmung besitzt, bei dem die Menge des von der Atemgasquelle zugeführten Atemgases über einen Regler (1) einstellbar ist, bei dem die maximale Eintauchtiefe des Steigrohrs (14) den maximalen inspiratorischen Beatmungsdruck bestimmt, und bei dem die Verschlussdauer der Ausatemöffnung Atemfrequenz und Atemphase bestimmt, dadurch gekennzeichnet, dass zwei Beatmungsstufen vorgesehen sind, deren erste Beatmungsstufe durch das Wasserschloss (2) gebildet ist und deren zweite Beatmungsstufe zuschaltbar ist und durch ein mit dem Wasserschloss (2) der ersten Beatmungsstufe in Reihe schaltbares zweites Wasserschloss (32) oder ein Druckbegrenzungsventil gebildet ist.

8. Beatmungsgerät nach Anspruch 7, dadurch gekennzeichnet, dass zur Bildung weiterer Beatmungsstufen weitere Wasserschlösser in Reihe zuschaltbar sind.

9. Beatmungsgerät nach Anspruch 7 oder 8, dadurch gekennzeichnet, dass die Verbindungsleitung (38) zwischen der Zuführöffnung (39) eines zuschaltbaren Wasserschlosses (32) und der Abführöffnung (37) des vorhergehenden Wasserschlosses (2) starr ist.

10. Beatmungsgerät nach Anspruch 9, dadurch gekennzeichnet, dass die Verbindungsleitung (38) in der Befestigung (9) für die Wasserschlösser (2, 32) und den Regler (1) ausgebildet ist.

11. Beatmungsgerät nach einem der Ansprüche 7 bis 10, dadurch gekennzeichnet, dass zumindest das Wasserschloss (2) der ersten Beatmungsstufe zwei Auslassöffnungen (13, 37, 41, 42) aufweist, deren eine zur Verbindung mit dem Wasserschloss (32) der nächsten Beatmungsstufe dient, wobei deren andere zur Zuschaltung dieses Wasserschlosses (32) verschliessbar ist.

12. Beatmungsgerät nach Anspruch 11, dadurch gekennzeichnet, dass die andere Auslassöffnung (13, 41) nur verschliessbar ist, wenn der maximale Druck im Wasserschloss (2, 32) erreicht ist.

13. Beatmungsgerät nach Anspruch 11 oder 12, gekennzeichnet durch einen Stöpsel (49) zum Verschliessen der anderen Auslassöffnung (13, 41).

14. Beatmungsgerät nach Anspruch 13, dadurch gekennzeichnet, dass der Stöpsel (49) fest mit dem Steigrohr (14, 44) des Wasserschlosses (2, 32) verbunden ist.

15. Beatmungsgerät nach einem der Ansprüche 7 bis 14, dadurch gekennzeichnet, dass die Zufuhröffnung (39) zumindest eines schaltbaren Wasserschlosses (32) in den Innenraum einer Hülse (43) führt, die das in ihr verschiebbar geführte und nach aussen abgedichtete Steigrohr (44) dieses Wasserschlosses (32) umgibt, wobei im Steigrohr (44) eine Öffnung (45) vorgesehen ist, die über den gesamten dem Einstellbereich entsprechenden Verschiebungsbereich des Steigrohrs (44) innerhalb der Hülse (43) mündet.

16. Beatmungsgerät nach Anspruch 15, dadurch gekennzeichnet, dass bei Verwendung als Wasserschloss (2) der ersten Beatmungsstufe ein atemgasführender Schlauch (24) und ein zum Patientenadapter (4) führender Schlauch (25) starr am Gehäuse (10, 11) befestigt und mit einer Zuführöffnung verbunden sind.

17. Beatmungsgerät nach einem der Ansprüche 4 bis 16, dadurch gekennzeichnet, dass die Drosseleinrichtung (6') fest mit dem Patientenadapter (4) verbunden ist.

18. Beatmungsgerät nach Anspruch 17, dadurch gekennzeichnet, dass die Drosseleinrichtung (6') mit dem Patientenadapter (4) einstückig ist.

19. Beatmungsgerät nach einem der Ansprüche 4 bis 18, dadurch gekennzeichnet, dass die verschliessbare Öffnung des Patientenadapters (4) aus einem Rohr (19) besteht, dessen Querschnitt zyklisch zwischen einem Maximalwert und dem Minimalwert Null verstellbar ist.

20. Beatmungsgerät nach Anspruch 19, gekennzeichnet durch eine an die Querschnittsabmessungen des Rohrs (19) angepasste Kugel (20) mit einer Abplattung (30) in dem Rohr (19), die zyklisch um eine zur Abplattung (30) im wesentlichen paraallele Achse (21) drehbar ist.

21. Beatmungsgerät nach Anspruch 19 oder 20, dadurch gekennzeichnet, dass die Kugel (20) von einem Motor mit regelbarer oder einstellbarer Drehzahl drehbar ist.

22. Beatmungsgerät nach Anspruch 1 oder 7, gekennzeichnet durch eine Beatmungsdruckwerten entsprechende Skala (26, 51) an dem Steigrohr (14, 44) bzw. dem Gefäss (10, 35) des Wasserschlosses (2, 32).

23. Beatmungsgerät nach Anspruch 22, dadurch gekennzeichnet, dass bei skalierten Steigrohren (14, 44) die Steigrohre (14, 44) der verschiedenen Beatmungsstufen derart unterschiedlich ausgebildet sind, dass sie nur bei dem Wasserschloss (2, 32) der jeweiligen Beatmungsstufe verwendbar sind.

24. Beatmungsgerät nach Anspruch 23, dadurch gekennzeichnet, dass die skalierten Steigrohre (14, 44) der verschiedenen Beatmungsstufen unterschiedliche Querschnitte haben.

25. Beatmungsgerät nach Anspruch 1 oder 7, dadurch gekennzeichnet, dass das T-Verbindungsstück (16) mit dem Steigrohr (14) im Wasserschloss (2) starr verbunden ist.

26. Beatmungsgerät nach Anspruch 7 und 8, gekennzeichnet durch die Verwendung als Wasservakuummeter und -regler, wobei die letzte Beatmungsstufe als erste Saugstufe etc. und die erste Beatmungsstufe als letzte Saugstufe unter sinngemässem Tauschen von Zuführ- und Abführöffnungen dienen und Vakuumquelle und zu evakuierender Raum mit der Entlüftungsöffnung der letzten Beatmungsstufe verbunden sind.

**Claims**

1. Apparatus for artificial breathing for reanimation of a newborn, having a water column (2) as an inhalation valve with a T-connector element (16) connected to the riser tube (14) of the water column (2), through which the water column (2) is connected through breathing hoses (24, 25) with a breathing gas source and with a patient adapter (4) which includes a closable free opening (5) for exhalation standing in communication with the atmosphere, wherein the quantity of the breathing gas introduced from the breathing gas source is adjustable by a regulator (1), wherein the maximum immersion depth of the riser tube (14) determines the maximum inspiratory breathing pressure, and wherein the closing duration of the exhalation opening (5) determines the breathing frequency and breathing phase, characterized in that there are provided two breathing stages the first stage thereof consisting of said water column (2) and said second stage is connectable thereto and is consisting of a choke device (6) arranged in the vent opening (13) of the water column (2) of said first breathing stage whereby the cross-section of the choke device (6) as well as the quantity of the breathing gas introduced from the breathing gas source determine the value of the inspiratory breathing pressure.

2. Breathing apparatus according to claim 1, characterized by a safety valve member (3) in said second breathing stage, through which the inspiratory breathing pressure is limitable to a maximum value.

3. Breathing apparatus according to claim 2, characterized in that the safety valve member comprises a liquid manometer of the U-tube-type.

4. Apparatus for artificial breathing for reanimation of a newborn, having a water column (2) as an inhalation valve with a T-connector element (16) connected to the riser tube (14) of the water column (2), through which the water column (2) is connected through breathing hoses (24, 25) with a breathing gas source and with a patient adapter (4) which includes a closable free opening (5) for exhalation standing in communication with the atmosphere, wherein the quantity of the breathing gas introduced from the breathing gas source is adjustable by a regulator (1), wherein the maximum immersion depth of the riser tube (14) determines the maximum inspiratory breathing pressure, and wherein the closing duration of the exhalation opening (5) determines the breathing frequency and breathing phase, characterized in that within the exhalation opening (5, 23) of the patient adapter (4) for generating the end expiratory breathing pressure choke device (6, 6') is connectable wherein the cross-section of said connectable choke device (6, 6') as well as the quantity of the breathing gas introduced from the breathing gas source determine the value of the end expiratory breathing pressure.

5. Breathing apparatus according to anyone of claims 1 to 4, characterized in a plugable single choke device (6).

6. Breathing apparatus according to anyone of claims 1 to 4, characterized in that as said choke device (6) there is arranged a choke device (6) adjustable in cross-section whereby the value of the corresponding breathing pressures becomes independent of the quantity of the breathing gas introduced from the breathing gas source.

7. Apparatus for artificial breathing for reanimation of a newborn, having a water column (2) as an inhalation valve with a T-connector element (16) connected to the riser tube (14) of the water column (2), through which the water column (2) is connected through breathing hoses (24, 25) with a breathing gas source and with a patient adapter (4) which includes a closable free opening (5) for exhalation standing in communication with the atmosphere, wherein the quantity of the breathing gas introduced from the breathing gas source is adjustable by a regulator (1), wherein the maximum immersion depth of the riser tube (14) determines the maximum inspiratory breathing pressure, and wherein the closing duration of the exhalation opening (5) determines the breathing frequency and breathing phase, characterized in that there are provided two breathing stages, the first stage thereof consisting of said water column (2) and said second stage is connectable therein and is consisting of a further water column (32) which is connectable in series with the water column (2) of the first breathing stage or a pressure relief valve.

8. Breathing apparatus according to claim 7, characterized in that for forming further breathing stages, further water columns are connectable in series.

9. Breathing apparatus according to claim 7 or 8, characterized in that the connecting conduit (38) between the inlet opening (39) of a thereto connectable water column (32) and the discharge opening (37) of the preceding water column (2) is rigid.

10. Breathing apparatus according to claim 9, characterized in that the connecting conduit (38) is formed within the fastening means (9) for the water columns (2, 32) and the regulator (1).

11. Breathing apparatus according to anyone of claims 7 to 10, characterized in that at least the water column (2) of the first breathing stage comprises two outlet openings (13, 37; 41, 42), one of which serves for connection with the water column (32) of the subsequent breathing stage and wherein the other of which is closable for connecting this water column (32).

12. Breathing apparatus according to claim 11, characterized in that said other outlet opening (13, 41) is only closable when the maximum pressure is reached in the water column (2, 32).

13. Breathing apparatus according to claim 11 or 12, characterized by a stopper (49) for closing said other outlet opening (13, 41).

14. Breathing apparatus according to claim 13, characterized in that said stopper (49) is fixedly connected with said riser tube (14, 44) of said water column (2, 32).

15. Breathing apparatus according to anyone of claims 7 to 14, characterized in that the inlet opening (39) of a least one connectable water column (32) leads into the inner space of a sleeve (43) which encompasses a therein displacedly guided and externally sealed riser tube (44) of this water column (32), wherein an opening (45) is provided in said riser tube (44) which opens within said sleeve (43) over the entire range of displacement of said riser tube (44) corresponding to the adjustment range.

16. Breathing apparatus according to claim 15, characterized in that during utilization as the water column (2) of said first breathing stage, a breathing gasconveying hose (24) and a hose (25) leading to the patient adapter (4) are rigidly fastened to the housing (10, 11) and are connected with an inlet opening.

17. Breathing apparatus according to anyone of claims 4 to 16, characterized in that said choke device (6') is fixedly connected with the patient adapter (4).

18. Breathing apparatus according to claim 17, characterized in that said choke device (6') is integral with the patient adapter (4).

19. Breathing apparatus according to anyone of claims 4 to 18, characterized in that the closable opening of said patient adapter (4) consists of a tube (19) whose cross-section is cyclically adjustable between a maximum value and a zero minimum value.

20. Breathing apparatus according to claim 19, characterized by a ball (20) having a flattening (30) being in the tube (19) and conformed to the cross-sectional dimensions of the tube (19), which ball (20) is cyclically rotatable about an axis (21) essentially parallel to the flattening (30).

21. Breathing apparatus according to claim 19 or 20, characterized in that said ball (20) is rotatable by a motor with a controllable or adjustable rotational speed.

22. Breathing apparatus according to claim 1 or 7, characterized by a scale (26, 51) corresponding to the breathing pressure values on said riser tube (14, 44) or the vessel (10, 35) or said water column (2, 32).

23. Breathing apparatus according to claim 22, characterized in that with scaled riser tubes (14, 44), the riser tubes (14, 44) of the different breathing stages are so differently constructed that they are only employable at the water column (2, 32) of the respective breathing stage.

24. Breathing apparatus according to claim 23, charecterized in that the riser tubes (14, 44) of the respective breathing stages have differing cross-sections.

25. Breathing apparatus according to claim 10 or 7, characterized in that the T-connector element (16) is rigidly connected with the riser tube (14) in the water column (2).

26. Breathing apparatus according to claim 7 or 8, characterized by the utilization as watertype vacuum meter and controller, wherein the last breathing stage serves as the first suction stage, etc., and the first breathing stage serves as the last suction stage under correspondingly changing inlet and discharge openings, and a vacuum source and a space to be evacuated are connected with the vent opening of the last breathing stage.

**Revendications**

1. Appareil de respiration artificielle pour la réanimation de nouveau-nés, comportant une cheminée d'équilibre hydraulique (2) constituant une soupape d'inspiration, un raccord en té (16) relié au tube ascendant (14) de la cheminée (2), celle-ci étant reliée par le té (16), à une source de gaz respiratoire et à un adaptateur (4) pour le patient par l'intermédiaire de tuyaux de respiration (24, 25), cet adaptateur ayant une ouverture libre (5), qui est reliée à l'atmosphère, peut être fermée et est destinée à l'expiration, pour lequel la quantité de gaz respiratoire amené de la source de gaz respiratoire est réglable par un régulateur (1); et pour lequel la profondeur maximale de plongée du tube ascendant (14) détermine la pression inspiratoire maximale et la durée de fermeture de l'ouverture d'expiration (5) la fréquence respiratoire et le cycle respiratoire, caractérisé en ce qu'il est prévu deux étages respiratoires, dont le premier est formé par la cheminée d'équilibre hydraulique (2), tandis que le deuxième est raccordable et formé par un dispositif d'étranglement (6), placé dans l'ouverture (13) de purge d'air de la cheminée d'équilibre (2) du premier étage respiratoire, la section transversale du dispositif d'étranglement (6) aussi bien que la quantité de gaz respiratoire amenée de la source de gaz respiratoire déterminant la valeur de la pression inspiratoire.

2. Appareil de respiration artificielle suivant la revendication 1, caractérisé par un organe (3) de soupape de sûreté, par lequel la pression inspiratoire est apte à être limitée à une valeur maximale.

3. Appareil de respiration artificielle suivant la revendication 2, caractérisé en ce que l'organe de soupape de sûreté est formé par un manomètre (3) à liquide, à la manière d'un tube en U.

4. Appareil de respiration artificielle pour la réanimation de nouveau-nés, comportant une cheminée d'équilibre hydraulique (2) constituant une soupape d'inspiration, un raccord en té (16) relié au tube ascendant (14) de la cheminée (2), celle-ci étant reliée par le té (16), à une source de gaz respiratoire et à un adaptateur (4) pour le patient par l'intermédiaire de tuyaux de respiration (24, 25), cet adaptateur ayant une ouverture libre (5), qui est reliée à l'atmosphère, peut être fermée et est destinée à l'expiration; pour lequel la quantité de gaz respiratoire amenée de la source de gaz respiratoire est réglable par un régulateur (1); pour lequel la profondeur maximale du tube de plongée ascendant (14) détermine la pression inspiratoire maximale et pour lequel la durée de fermeture de l'ouverture d'expiration (5) détermine la fréquence respiratoire et le cycle respiratoire, caractérisé en ce que, dans l'ouverture d'expiration (5, 23) de l'adaptateur pour le patient (4), est raccordé un dispositif d'étranglement (6, 6') pour produire la pression de fin d'expiration, cependant que la section transversale du dispositif d'étranglement raccordable (6, 6') aussi bien que la quantité de gaz respiratoire amenée de la source de gaz respiratoire, déterminent la valeur de la pression de fin d'expiration.

5. Appareil de respiration artificielle suivant l'une des revendications 1 à 4, caractérisé par un dispositif d'étranglement (6) unique, dont l'enfichage est commutable.

6. Appareil de respiration artificielle suivant l'une des revendications 1 à 4, caractérisé en ce que, comme dispositif d'étranglement raccordable (6), on utilise un dispositif d'étranglement (6) à section transversale réglable, la valeur des pressions respiratoires correspondantes devenant indépendante de la quantité de gaz respiratoire amenée de la source de gaz respiratoire.

7. Appareil de respiration artificielle pour la réanimation de nouveau-nés, comportant une cheminée d'équilibre hydraulique (2) constituant une soupape d'inspiration, un raccord en té (16') relié au tube ascendant (14) de la cheminée (2), celle-ci étant reliée par le té (16'), à une source de gaz respiratoire et à un adaptateur (4) pour le patient par l'intermédiaire de tuyaux de respiration (24, 25), cet adaptateur ayant une ouverture libre, qui est reliée à l'atmosphère, peut être fermée et est destinée à l'expiration; pour lequel la quantité de gaz respiratoire amenée de la source de gaz respiratoire est réglable par un régulateur (1); pour lequel la profondeur maximale du tube de plongée ascendant (14) détermine la pression inspiratoire maximale et pour lequel la durée de fermeture de l'ouverture d'expiration détermine la fréquence respiratoire et le cycle respiratoire, caractérisé en ce qu'il est prévu deux étages respiratoires, dont le premier est formé par la cheminée d'équilibre hydraulique (2), tandis que le deuxième est raccordable et formée par une deuxième cheminée d'équilibre hydraulique (32), montée en série avec la cheminée d'équilibre (2) du premier étage respiratoire, ou formée par une soupape de limitation de pression.

8. Appareil de respiration artificielle suivant la revendication 7, caractérisé en ce que, pour former d'autres étages respiratoires, d'autres cheminées d'équilibre hydraulique sont raccordables en série.

9. Appareil de respiration artificielle suivant la revendication 7 ou 8, caractérisé en ce que la conduite de liaison (38) entre l'ouverture d'arrivée (39) d'une cheminée d'équilibre hydraulique (32) raccordable et l'ouverture d'évacuation (37) de la cheminée d'équilibre précédente (2) est rigide.

10. Appareil de respiration artificielle suivant la revendication 9, caractérisé en ce que la conduite de liaison (38) est formée dans une fixation (9) pour les cheminées d'équilibre hydraulique (2, 32) et pour le régulateur (1).

11. Appareil de respiration artificielle suivant l'une des revendications 7 à 10, caractérisé en ce qu'au moins la cheminée d'équilibre (2) du premier étage respiratoire présente deux ouvertures de sortie (13, 37, 41, 42), dont l'une sert à la liaison avec la cheminée d'équilibre (32) de l'étage respiratoire suivant, tandis que l'autre peut se fermer pour le raccordement de cette cheminée d'équilibre (32).

12. Appareil de respiration artificielle suivant la revendication 11, caractérisé en ce que l'autre ouverture de sortie (13, 41) ne peut se fermer que si la pression maximale est atteinte dans la cheminée d'équilibre hydraulique (2, 32).

13. Appareil de respiration artificielle suivant la revendication 11 ou 12, caractérisé par un bouchon (49), servant à fermer l'autre ouverture de sortie (13, 41).

14. Appareil de respiration artificielle suivant la revendication 13, caractérisé en ce que le bouchon (49) est fixé au tube ascendant (14, 44) de la cheminée d'équilibre (2, 32).

15. Appareil de respiration artificielle suivant une des revendications 7 à 14, caractérisé en ce que l'ouverture d'arrivée (39) d'au moins une cheminée d'équilibre (32) raccordable mène à l'intérieur d'un manchon (43), qui entoure le tube ascendant (44) de cette cheminée d'équilibre (32), le tube (44) étant logé mobile dans le manchon et étanche vers l'extérieur, cependant qu'il est prévu dans ce tube ascendant (44) une ouverture (45), qui débouche au-dessus de l'ensemble de la zone de déplacement du tube ascendant (44), correspondant à la zone de réglage, à l'intérieur du manchon (43).

16. Appareil de respiration artificielle suivant la revendication 15, caractérisé en ce que, dans le cas de l'utilisation comme cheminée d'équilibre (2) du premier étage respiratoire, un tuyau (24) à gaz respiratoire et un tuyau (25) menant à l'adaptateur (4) pour le patient sont fixés rigidement à l'enveloppe (10, 11) et reliés à une ouverture d'arrivée.

17. Appareil de respiration artificielle suivant une des revendications 4 à 16, caractérisé en ce que le dispositif d'étranglement (6') est fixé à l'adaptateur (4) pour le patient.

18. Appareil de respiration artificielle suivant la revendication 17, caractérisé en ce que le dispositif d'étranglement (6') est d'une pièce avec l'adaptateur (4) pour le patient.

19. Appareil de respiration artificielle suivant une des revendications 4 à 18, caractérisé en ce que l'ouverture – qui peut être fermée – de l'adaptateur (4) pour le patient est formée d'un tube (19), dont la section transversale est réglable cycliquement entre un maximum et le minimum égal à zéro.

20. Appareil de respiration artificielle suivant la revendication 19, caractérisé par une bille (20), adaptée aux dimensions de la section transversale du tuyau (19), comportant un méplat (30) et logée dans le tuyau (19), cette bille pouvant tourner cycliquement autour d'un axe (21) essentiellement parallèle au méplat (30).

21. Appareil de respiration artificielle suivant la revendication 19 ou 20, caractérisé en ce que la bille (20) peut être entraînée en rotation par un moteur à vitesse réglable.

22. Appareil de respiration artificielle suivant la revendication 1 ou 7, caractérisé par une échelle graduée (26, 51), correspondant à des valeurs de pressions respiratoires et disposée sur le tube ascendant (14, 44) et/ou sur le récipient (10, 35) de la cheminée d'équilibre hydraulique (2, 32).

23. Appareil de respiration artificielle suivant la revendication 22, caractérisé en ce que, dans le cas de tubes ascendants (14, 44) gradués, les tubes ascendants (14, 44) des différents étages respiratoires sont réalisés différemment, de manière à n'être utilisables qu'avec la cheminée d'équilibre (2, 32) de l'étage respiratoire correspondant.

24. Appareil de respiration artificielle suivant la revendication 23, caractérisé en ce que les tubes ascendants gradués (14, 44) des différents étages respiratoires ont différents diamètres transversaux.

25. Appareil de respiration artificielle suivant la revendication 1 ou 7, caractérisé en ce que le raccord en té (16) est relié rigidement au tube ascendant (14) qui est dans la cheminée d'équilibre (2).

26. Appareil de respiration artificielle suivant les revendications 7 et 8, caractérisé par une utilisation comme mesureur de vide d'eau et régulateur de vide d'eau, le dernier étage respiratoire servant de premier étage d'aspiration, etc. et le premier étage respiratoire servant de dernier étage d'aspiration, les ouvertures d'arrivée et d'évacuation étant judicieusement permutées, cependant que la source de vide et l'espace à évacuer sont reliés à l'ouverture de purge d'air du dernier étage respiratoire.

FIG. 1

FIG. 2

FIG. 3b

FIG. 3a

FIG. 4a          FIG. 4b

0 033 732

FIG. 5

FIG. 6